# EUROPÄISCHE PATENTSCHRIFT

(11) **EP 2 415 679 B1**
(45) Veröffentlichungstag und Bekanntmachung des Hinweises auf die Patenterteilung: **03.04.2013**
(21) Anmeldenummer: 11005846.8
(22) Anmeldetag: 16.07.2011
(51) Int. Cl.: B65C 9/42, B65H 7/12, G01N 29/11, G01N 33/34, G01N 29/28

(54) **Ultraschallsensor**
Ultrasound sensor
Capteur d'ultrasons

(30) Priorität: 05.08.2010 DE 202010011113 U
(43) Veröffentlichungstag der Anmeldung: 08.02.2012
(73) Patentinhaber: Leuze electronic GmbH + Co. KG, 73277 Owen/Teck (DE)
(72) Erfinder: Klaiber, Jörg, 71157 Hildrizhausen (DE); Merk, Thomas, 73240 Wendlingen (DE); Patz, Jürgen, 72660 Beuren (DE)
(74) Vertreter: Ruckh, Rainer Gerhard

(56) Entgegenhaltungen:
- EP-A1- 2 116 473
- EP-A1- 2 116 474
- EP-A2- 0 167 010

## Beschreibung

Die Erfindung betrifft einen Ultraschallsensor gemäß dem Oberbegriff des Anspruchs 1.

Ein derartiger Ultraschallsensor ist aus der DE 10 2008 023 185 A1 bekannt. Dieser Ultraschallsensor dient zur Erfassung von Objekten in einem Überwachungsbereich und umfasst einen Ultraschallwellen emittierenden Sender, einen Ultraschallwellen empfangenden Empfänger, eine Auswerteeinheit zur Generierung eines Objektfeststellungssignals in Abhängigkeit der Empfangssignale am Ausgang des Empfängers und ein zwei Gabelarme aufweisendes Sensorgehäuse. Die Gabelarme begrenzen den Überwachungsbereich. In einem ersten Gabelarm ist eine Austrittsfläche vorgesehen, durch welche die Ultraschallwellen in den Überwachungsbereich geführt sind. Im zweiten Gabelarm ist eine Eintrittsfläche vorgesehen, durch welche Ultraschallwellen aus dem Überwachungsbereich in das Sensorgehäuse geführt sind. An der Eintrittsfläche ist eine Blende vorgesehen.

Durch die Anbringung der empfangsseitigen Blende kann der Störeinfluss von sich zwischen den Gabelarmen ausbildenden, stehenden Wellen signifikant reduziert werden, wodurch eine beträchtliche Erhöhung der Nachweisempfindlichkeit des Ultraschallsensors erzielt wird.

Diese Nachweisempfindlichkeit kann noch weiter dadurch gesteigert werden, indem auch sendeseitig eine Blende vorgesehen wird.

Mit dem Ultraschallsensor werden generell unterschiedliche Objekte detektiert, insbesondere verschiedene, auf Trägermaterial angebrachte Etiketten oder andere mehrlagige Objektstrukturen.

Je nach Ausbildung der Objekte, insbesondere bei der Detektion von Etiketten unterschiedlicher Größen, ist es zur Erzielung einer hinreichenden Nachweissicherheit notwendig, den Strahlquerschnitt der Ultraschallwellen an die zu detektierenden Objektstrukturen anzupassen.

Eine Möglichkeit der Anpassung besteht darin, mit unterschiedlichen Blenden, das heißt mit Blenden, die verschiedene Blendenöffnungen zur Strahlformung der Ultraschallwellen aufweisen, zu arbeiten. Nachteilig hierbei ist jedoch, dass für jede spezifische Vorgabe des Strahlquerschnitts der Ultraschallwellen verschiedene Blenden eingesetzt werden müssen. Dies führt zu einer unerwünscht großen Teileanzahl zur Ausbildung des Ultraschallsensors. Dieser Nachteil wird dann noch verstärkt, wenn bei dem Ultraschallsensor sowohl sendeseitig an der Austrittsfläche als auch empfangsseitig an der Eintrittsfläche Blenden vorgesehen sind, so dass dann auf beiden Seiten die Blenden ausgetauscht werden müssen.

Die EP 2 116 473 A1 betrifft einen Ultraschallsensor der zur Erfassung von Objekten in einem Überwachungsbereich dient. Der Ultraschallsensor umfasst einen Ultraschallwellen emittierenden Sender, einen Ultraschallwellen empfangenden Empfänger, eine Auswerteeinheit zur Generierung eines Objektfeststellungssignals in Abhängigkeit der Empfangssignale am Ausgang des Empfängers und ein zwei Gabelarme aufweisendes Sensorgehäuse. Die Gabelarme begrenzen den Überwachungsbereich. In einem ersten Gabelarm ist eine Austrittsfläche vorgesehen, durch welche die Ultraschallwellen in den Überwachungsbereich geführt sind. Im zweiten Gabelarm ist eine Eintrittsfläche vorgesehen, durch welche Ultraschallwellen aus dem Überwachungsbereich in das Sensorgehäuse geführt sind. An der Eintrittsfläche ist eine Blende vorgesehen.

Der Erfindung liegt die Aufgabe zugrunde, einen Ultraschallsensor der eingangs genannten Art so auszubilden, dass mit diesem bei geringem konstruktiven Aufwand unterschiedliche Objekte detektiert werden können.

Zur Lösung dieser Aufgabe sind die Merkmale des Anspruchs 1 vorgesehen. Vorteilhafte Ausführungsformen und zweckmäßige Weiterbildungen der Erfindung sind in den Unteransprüchen beschrieben.

Der erfindungsgemäße Ultraschallsensor dient zur Erfassung von Objekten in einem Überwachungsbereich, mit einem Sensorgehäuse, einem Sender, der Ultraschallwellen emittiert, welche über eine erste Durchtrittsfläche des Sensorgehäuses in den Überwachungsbereich geführt sind. Der Empfänger empfängt Ultraschallwellen, die aus dem Überwachungsbereich kommend über eine zweite Durchtrittsfläche des Sensorgehäuses geführt sind. Eine Auswerteeinheit dient zur Generierung eines Objektfeststellungssignals in Abhängigkeit der Empfangssignale des Empfängers. Die Objektdetektion erfolgt nach dem Transmissionsprinzip. Ein Blendenkörper ist in unterschiedlicher Einstellposition am Sensorgehäuse anbringbar. In den einzelnen Einstellpositionen sind unterschiedliche Blendenöffnungen des Blendenkörpers vor einer Durchtrittsfläche positioniert.

Mit den unterschiedlichen, beziehungsweise variablen Blendenöffnungen des Blendenkörpers kann der Strahlquerschnitt der Ultraschallwellen einfach und schnell verändert werden, wodurch der Ultraschallsensor flexibel an unterschiedliche Applikationen, insbesondere an die Detektion unterschiedlicher Größen, angepasst werden kann. Besonders vorteilhaft ist dabei, dass mit einem Blendenkörper mehrere unterschiedliche Blendenöffnungen zur Strahlformung der Ultraschallwellen bereitgestellt werden können, so dass die Teileanzahl des Ultraschallsensors gering gehalten werden kann.

Durch eine empfangsseitige Anordnung des Blendenkörpers mit einer an die jeweilige Applikation angepasster Blendenöffnung können Störeinflüsse reduziert werden. Insbesondere können durch die Blendenöffnung Störeinflüsse durch stehende Wellen oder von Teilen des Sensorgehäuses reflektierten Ultraschallwellen eliminiert oder zumindest reduziert werden.

Durch eine sendeseitige Anordnung des Blendenkörpers kann eine geeignete Blendenöffnung des Blendenkörpers gewählt werden, um einen optimalen, an die Größen der zu detektierenden Objekte angepassten Strahlquerschnitt der in den Überwachungsbereich geführten Ultraschallwellen zu erhalten.

Prinzipiell kann der Ultraschallsensor ein mehrteiliges Sensorgehäuse aufweisen, wobei in einem Gehäuseteil der Sender und in einem zweiten Gehäuseteil der Empfänger mit der Auswerteeinheit integriert sind. In diesem Fall werden Blendenkörper für die einzelnen Gehäuseteile des Ultraschallsensors bereitgestellt, die bevorzugt an die Formen der Gehäuseteile angepasst sind.

Besonders vorteilhaft ist das Sensorgehäuse des Ultraschallsensors einteilig ausgebildet und dient zur Aufnahme räumlicher Komponenten des Ultraschallsensors.

Insbesondere ist das Sensorgehäuse gabelförmig ausgebildet und weist zwei parallel in Abstand zueinander verlaufende Gabelarme auf. In einem Gabelarm ist der Sender und im anderen Gabelarm der Empfänger angeordnet.

Die Blendenkörper sind dann so ausgebildet, dass diese passgenau an den einzelnen Gabelarmen angebracht werden können.

Gemäß einer ersten Variante der Erfindung sind in einem Blendenkörper mehrere, in Abstand zueinander liegende Blendenöffnungen vorgesehen. In den einzelnen Einstellpositionen ist der Blendenkörper in verschiedenen Lagen am Sensorgehäuse fixiert, so dass jeweils unterschiedliche Blendenöffnungen vor einer Durchtrittsfläche positioniert sind.

Um eine gewünschte Blendenöffnung vor der Durchtrittsfläche des Sensorgehäuses zu positionieren, wird der Blendenkörper in einer bestimmten Lage auf dem Sensorgehäuse aufgerastet. Soll die Blendenöffnung gewechselt werden, muss der Blendenkörper lediglich in einer anderen Position aufgerastet werden. Die Variation von Blendenöffnungen zur Strahlformung der Ultraschallsensoren kann durch das unterschiedliche Aufrasten des Blendenkörpers sehr einfach und schnell durchgeführt werden.

Gemäße einer zweiten Variante der Erfindung weist der Blendenkörper nur eine Blendenöffnung auf, deren Form und/oder Größe mittels eines Verstellmechanismus einstellbar ist.

Die Variation der Blendenöffnung kann damit allein durch eine Betätigung des Verstellmechanismus erfolgen, wobei der Blendenkörper bei unterschiedlichen Einstellungen der Blendenöffnung in unveränderter Lage am Sensorgehäuse fixiert ist, das heißt der Blendenkörper muss zur Variation der Blendenöffnung nicht vom Sensorgehäuse abgenommen werden.

Besonders vorteilhaft ist mittels des Verstellmechanismus die Form und/oder Größe der Blendenöffnung stufenlos einstellbar.

Gemäß einer besonders zweckmäßigen Ausgestaltung ist im Blendenkörper ein Blendenloch eingearbeitet, welches zur Ausbildung einer einstellbaren Blendenöffnung mit Blendenteilen, die mittels des Verstellmechanismus einstellbar sind, mit variablem Überdeckungsgrad abdeckbar ist. Die Blendenteile sind mittels des Verstellmechanismus in gegenläufigen Richtungen verschiebbar gelagert. Der Verstellmechanismus weist zwei auf einer Spindel angeordnete gegenläufige Gewinde zur Verstellung der Blendenteile auf. Der Verstellmechanismus weist eine Stellschraube als Betätigungselement auf.

Der so ausgebildete Verstellmechanismus weist einen einfachen, robusten Aufbau auf und ermöglicht eine stufenlose Einstellung der Blendenöffnung.

Die Erfindung wird im Folgenden anhand der Zeichnungen erläutert. Es zeigen:
- Figur 1:: In einem Sensorgehäuse integrierter Ultraschallsensor mit einer ersten Ausführungsform eines Blendenkörpers in einer ersten Einstellposition.
- Figur 2:: Ultraschallsensor gemäß Figur 1 mit dem Blendenkörper in einer zweiten Einstellposition.
- Figur 3:: Einzeldarstellung des Blendenkörpers gemäß den Figuren 1 und 2.
- Figur 4:: Ultraschallsensor mit einer zweiten Ausftihrungsform eines Blendenkörpers.
- Figur 5:: Einzeldarstellung des Blendenkörpers gemäß Figur 5 mit einer ersten Einstellung einer Blendenöffnung.
- Figur 6:: Einzeldarstellung des Blendenkörpers gemäß Figur 5 mit einer zweiten Einstellung einer Blendenöffnung.

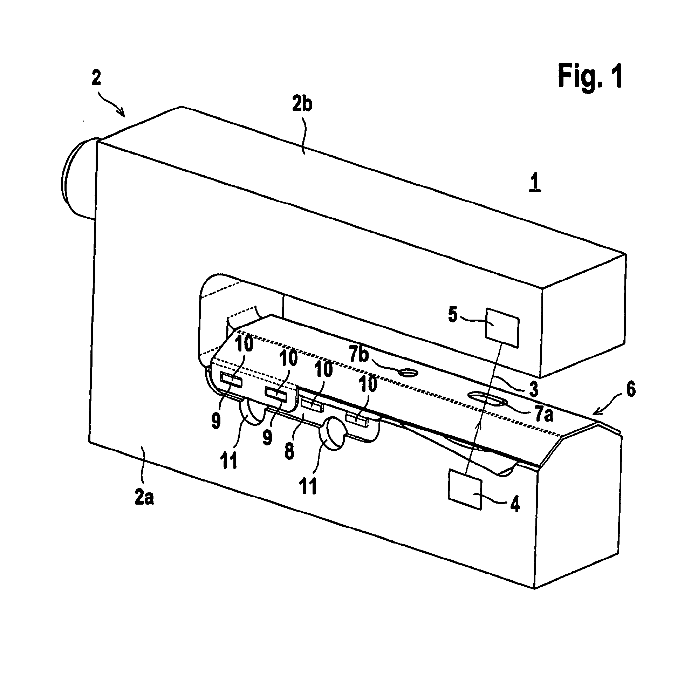

Figur 1 zeigt ein Ausführungsbeispiel eines Ultraschallsensors 1 zur Erfassung von Objekten in einem Überwachungsbereich. Der Ultraschallsensor 1 kann insbesondere zur Detektion von mehrlagigen Objektstrukturen eingesetzt werden. Beispielsweise können mit dem Ultraschallsensor 1 Etiketten auf Trägermaterialien detektiert werden.

Die Komponenten des Ultraschallsensors 1 sind in einem einteiligen Sensorgehäuse 2 integriert. Das Sensorgehäuse 2 ist gabelförmig ausgebildet und weist zwei parallel in Abstand zueinander verlaufende Gabelarme 2a, 2b auf. Der Ultraschallsensor 1 weist einen Ultraschallwellen 3 emittierenden Sender 4 und einen Ultraschallwellen 3 empfangenden Empfänger 5 auf. Im vorliegenden Fall emittiert der Sender 4 Ultraschallwellen 3 in Form von Sendepulspaketen mit definierten Pulsdauern und Pulspausen. Die Ultraschallwellen 3 sind in Figur 1 schematisch mit einem Pfeil gekennzeichnet. Der Sender 4 ist im ersten Gabelarm 2a schräg gestellt angeordnet, so dass die Strahlrichtung der Ultraschallwellen 3 in einem Neigungswinkel zur Horizontalen und damit zu den Längsachsen der Gabelarme 2a, 2b verläuft. Der Sender 4 liegt dicht hinter einer Durchtrittsfläche des Gabelarms 2a, durch welche die vom Sender 4 emittierten Ultraschallwellen 3 in den Überwachungsbereich geführt sind. Der Empfänger 5 ist im zweiten Gabelarm 2b mit einer entsprechenden Neigung angeordnet. Der Empfänger 5 liegt dabei dicht hinter einer Durchtrittsfläche des Gabelarms 2a, durch welche die aus dem Überwachungsbereich kommenden Ultraschallwellen 3 zum Empfänger 5 geführt sind. Der Zwischenraum zwischen den Gabelarmen 2a, 2b bildet den Überwachungsbereich, innerhalb dessen Objekte erkannt werden.

Die Neigung der Strahlachse der Ultraschallwellen 3 verläuft in der von den Längsachsen der Gabelarme 2a, 2b aufgespannten Ebene und senkrecht zur Förderrichtung, in welcher die zu detektierenden Objekte durch den Zwischenraum zwischen den Gabelarmen 2a, 2b bewegt werden.

In dem Sensorgehäuse 2 ist eine nicht gesondert dargestellte Auswerteeinheit angeordnet, die einerseits zur Ansteuerung des Senders 4 und andererseits zur Auswertung der Empfangssignale des Empfängers 5 dient. Die Objektdetektion erfolgt dabei nach dem Transmissionsprinzip, indem im Empfänger 5 der ein Objekt durchsetzende Teil der Ultraschallwellen 3 registriert und in der Auswerteeinheit ausgewertet wird. In Abhängigkeit der Empfangssignale wird in der Auswerteeinheit ein Objektfeststellungssignal generiert. Insbesondere kann in der Auswerteeinheit durch eine Schwellwertbewertung ein binäres Schaltsignal generiert werden. Die beiden Schaltzustände des Schaltsignals geben im einfachsten Fall an, ob ein Objekt vorhanden ist oder nicht. Für den Fall, dass die Objekte von auf Trägermaterialien angeordneten Etiketten gebildet sind, geben die Schaltzustände an, ob eine Etikette auf dem Trägermaterial vorhanden ist oder nicht.

Wie aus Figur 1 ersichtlich, ist auf dem unteren Gabelarm 2a ein Blendenkörper 6 fixiert. Prinzipiell könnte ein entsprechender Blendenkörper 6 auf dem oberen Gabelarm 2b angebracht sein. Im vorliegenden Ausführungsbeispie! ist jedoch das Sensorgehäuse 2 so ausgebildet, dass allein am unteren Gabelarm 2a ein Blendenkörper 6 fixiert werden kann.

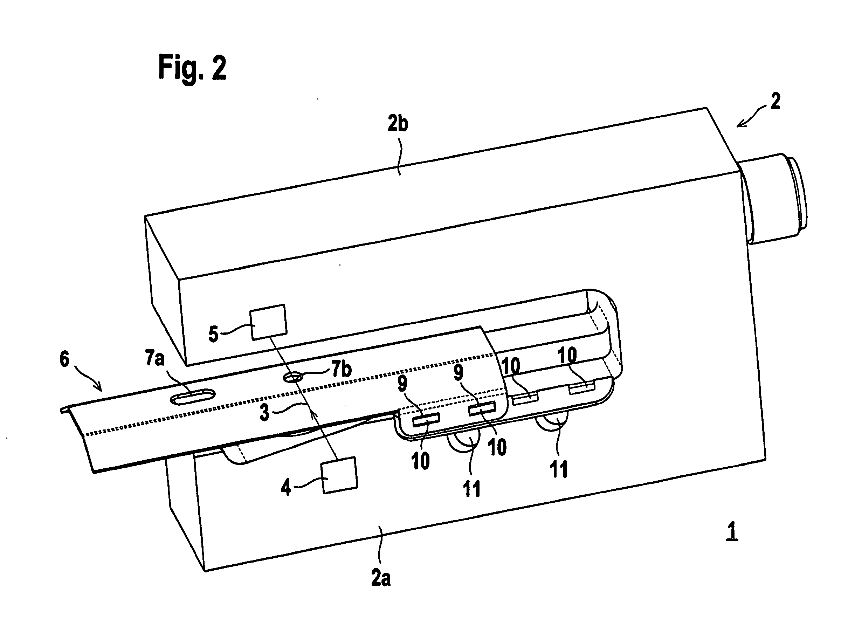

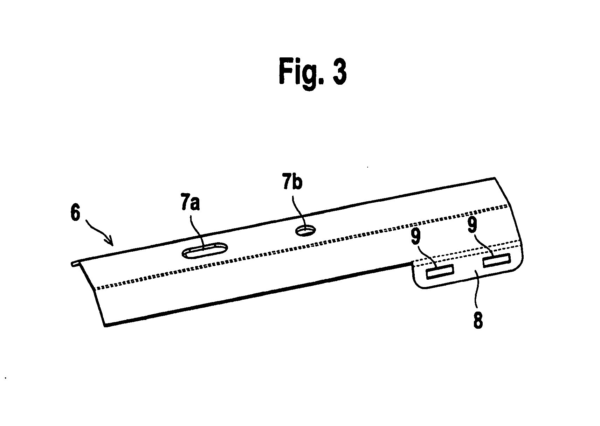

Figur 1 zeigt den Blendenkörper 6 in einer ersten Einstellposition am Sensorgehäuse 2. Figur 2 zeigt den Blendenkörper 6 in einer zweiten Einstellposition am Sensorgehäuse 2. Schließlich zeigt Figur 3 den Blendenkörper 6 in einer Einzeldarstellung.

Der Blendenkörper 6 besteht aus einem Formteil, dessen Kontur an die Kontur des Sensorgehäuses 2 angepasst ist. In dem Blendenkörper 6 sind zwei unterschiedliche, in Abstand zueinander liegende Blendenöffnungen 7a, 7b eingearbeitet. Die erste Blendenöffnung 7a weist eine langgestreckte Form auf, deren Enden abgerundet sind. Die zweite, kleinere Blendenöffnung 7b ist kreisförmig ausgebildet.

An der Unterseite des Blendenkörpers 6 sind zwei identische, gegenüberliegend angeordnete Befestigungslaschen 8 vorgesehen, in welche Aussparungen 9 eingearbeitet sind. Zur Befestigung des Blendenkörpers 6 am Gabelarm 2a wird der Blendenkörper 6 auf den Gabelarm 2a so aufgesetzt, dass die Aussparungen 9 in Rastnasen 10 am Gabelarm 2a einrasten.

Wie aus den Figuren 1 bis 3 ersichtlich, sind an jeder Befestigungslasche 8 zwei Aussparungen 9 vorhanden. Jedoch sind auf beiden Seiten des Gabelarms 2a vier Rastnasen 10 vorgesehen. Damit kann der Blendenkörper 6 in den beiden in den Figuren 1 und 2 dargestellten Einstellpositionen auf dem Gabelarm 2a aufgerastet werden. In der in Figur 1 dargestellten Einstellposition liegt die erste Blendenöffnung 7a vor der Durchtrittsfläche, die dem Sender 4 vorgeordnet ist. Damit erfolgt in dieser Einstellposition mit der Blendenöffnung 7a eine Strahlformung der Ultraschallwellen 3. Wie aus Figur 1 ersichtlich, schließt in dieser Einstellposition der Blendenkörper 6 bündig mit dem vorderen Rand des Gabelarms 2a ab.

In der in Figur 2 dargestellten Einstellposition liegt die zweite Blendenöffnung 7b vor der Durchtrittsfläche, die dem Sender 4 vorgeordnet ist. Damit erfolgt in dieser Einstellposition mit der Blendenöffnung 7b die Strahlformung der Ultraschallwellen 3. In dieser Einstellposition steht der Blendenkörper 6 über den vorderen Rand des Gabelarms 2a hervor.

Für einen Wechsel der Einstellposition wird der Blendenkörper 6 vom Gabelarm 2a abgelöst und dann bezüglich seiner Längsachse versetzt auf dem Gabelarm 2a aufgerastet. Um das Abnehmen des Blendenkörpers 6 vom Gabelarm 2a zu erleichtern, sind an den Seitenwänden des Gabelarms 2a ausmündende Aushebel-Öffnungen 11 vorgesehen. Durch Einstecken eines spitzen Gegenstands in eine dieser Aushebel-Öffnungen 11 wird der Blendenkörper 6 von den Gabelarmen 2a, 2b und die Rastverbindungen zwischen diesen Einheiten gelöst.

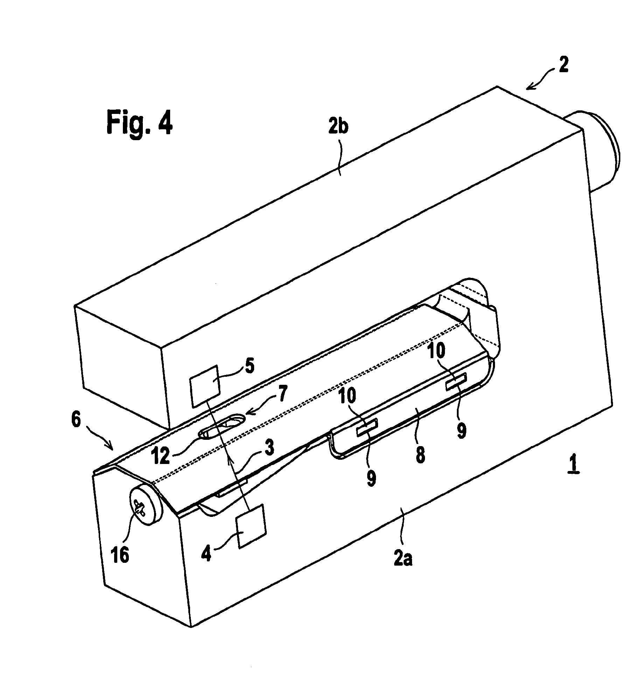

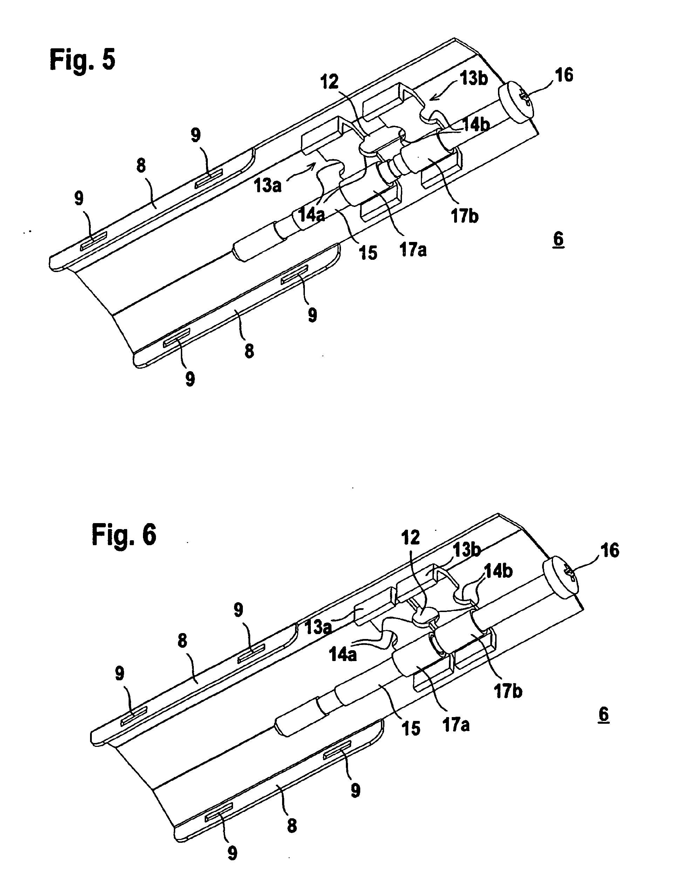

Figur 4 zeigt den Ultraschallsensor 1 der Figuren 1 und 2 mit einer zweiten Ausführungsform eines Blendenkörpers 6. Die Figuren 5 und 6 zeigen jeweils eine Draufsicht auf die Unterseite dieses Blendenkörpers 6 für unterschiedliche Blendeneinstellungen.

Analog zur Ausführungsform gemäß den Figuren 1 bis 3 besteht auch der Blendenkörper 6 gemäß den Figuren 4 bis 6 aus einem Blechteil, dessen Kontur an die Kontur des Gabelarms 2a angepasst ist. In weiterer Übereinstimmung mit der Ausführungsform der Figuren 1 bis 3 weist auch der Blendenkörper 6 gemäß den Figuren 4 bis 6 an seiner Unterseite zwei gegenüberliegend angeordnete Befestigungslaschen 8 auf, in welche Aussparungen 9 eingearbeitet sind. Zur Befestigung des Blendenkörpers 6 auf dem Gabelarm 2a werden wiederum die Aussparungen 9 des Blendenkörpers 6 in Rastnasen (10) an den Seitenwänden des Gabelarms 2a eingerastet.

Im Unterschied zur Ausführungsform gemäß den Figuren 1 bis 3 wird der Blendenkörper 6 gemäß den Figuren 4 bis 6 nur in einer festen Position auf dem Gabelarm 2a aufgerastet. Auch weist der Blendenkörper 6 der Figuren 4 bis 6 nicht mehrere beabstandet angeordnete Blendenöffnungen 7a, 7b auf, sondern nur eine Blendenöffnung 7. Die Blendeneinstellung erfolgt im vorliegenden Fall bei fest an dem Gabelarm 2a angeordneten Blendenkörper 6 durch eine Einstellung der Größe und Form der Blendenöffnung 7 mittels eines Verstellmechanismus.

Zur Ausbildung der Blendenöffnung 7 ist, wie aus den Figuren 4 bis 6 ersichtlich, in den Blendenkörper 6 ein langgestrecktes, an den längsseitigen Enden abgerundetes Blendenloch 12 eingearbeitet. Dieses Blendenloch 12 definiert die maximale Größe der Blendenöffnung 7.

Die Blendenöffnung 7a kann dadurch verkleinert werden, dass zwei Blendenteile 13a, 13b in zum Mittelpunkt des Blendenlochs 12 symmetrischer, gegenläufiger Verschieberichtung in den Bereich des Blendenlochs 12 eingeschoben werden, wodurch das Blendenloch 12 teilweise abgedeckt wird. Die Blendenteile 13a, 13b weisen dabei Ausnehmungen 14a, 14b auf, die an die Konturen der längsseitigen abgerundeten Enden des Blendenlochs 12 angepasst sind, so dass diese Konturen der Blendenöffnungen 7a, 7b bei Einschieben der Blendenteile 13a, 13b erhalten bleiben.

Der Verstellmechanismus zum Verschieben der Blendenteile 13a, 13b und damit zur Einstellung der Blendenöffnung 7 ist von einer am Blendenkörper 6 gelagerten Spindel 15 gebildet, die mittels einer an einem längsseitigen Ende des Blendenkörpers 6 zugänglich gelagerten Stellschraube 16 betätigt werden kann. Die Blendenteile 13a, 13b weisen hochzylindrische Führungen 17a, 17b mit Innengewinden auf, die jeweils ein Gewinde auf der Spindel 15 umschlie-βen. Die Gewinde sind gegenläufig ausgebildet. Wird daher die Stellschraube 16 betätigt, so werden die Blendenteile 13a, 13b je nach Betätigungsrichtung zusammen- oder auseinander gefahren, wodurch die Blendenöffnung 7 verkleinert oder vergrößert wird.

Damit kann durch Betätigen der Stellschraube 16 die Blendenöffnung 7 stufenlos vergrößert oder verkleinert werden. Figur 5 zeigt die Blendenöffnung 7 bei maximaler Größe, die dadurch erhalten ist, dass die Blendenteile 13a, 13b aus dem Bereich des Blendenlochs 12 herausgeschoben sind. Figur 6 zeigt die Blendenöffnung 7 maximaler Größe, die dadurch erhalten ist, dass die Blendenteile 13a, 13b gegeneinander gefahren sind und sich berühren, so dass die Blendenöffnung 7 durch die von den Ausnehmungen 14a, 14b der Blendenteile 13a, 13b ausgeformten Öffnungen gebildet ist. Bezugszeichenliste
- (1): Ultraschallsensor
- (2): Sensorgehäuse
- (2a): erster Gabelarm
- (2b): zweiter Gabelarm
- (3): Ultraschallwellen
- (4): Sender
- (5): Empfänger
- (6): Blendenkörper
- (7): Blendenöffnung
- (7a): erste Blendenöffnung
- (7b): zweite Blendenöffnung
- (8): Befestigungslasche
- (9): Aussparung
- (10): Rastnase
- (11): Aushebel-Öffnung
- (12): Blendenloch
- (13a): Blendenteil
- (13b): Blendenteil
- (14a): Ausnehmung
- (14b): Ausnehmung
- (15): Spindel
- (16): Stellschraube
- (17a): Führung
- (17b): Führung

## Patentansprüche

1. Ultraschallsensor (1) zur Erfassung von Objekten in einem Überwachungsbereich, mit einem Sensorgehäuse (2), einem Sender (4), der Ultraschallwellen (3) emittiert, welche über eine erste Durchtrittsfläche des Sensorgehäuses (2) in den Überwachungsbereich geführt sind, einem Empfänger (5), der Ultraschallwellen (3) empfängt, die aus dem Überwachungsbereich kommend über eine zweite Durchtrittsfläche des Sensorgehäuses (2) geführt sind, und mit einer Auswerteeinheit zur Generierung eines Objektfeststellungssignals in Abhängigkeit der Empfangssignale des Empfängers (5), wobei die Objektdetektion nach dem Transmissionsprinzip erfolgt, **dadurch gekennzeichnet, dass** wenigstens ein Blendenkörper (6) in unterschiedlichen Einstellpositionen am Sensorgehäuse (2) anbringbar ist, wobei in den einzelnen Einstellpositionen unterschiedliche Blendenöffnungen (7, 7a, 7b) des Blendenkörpers (6) vor einer Durchtrittsfläche positioniert sind.

2. Ultraschallsensor nach Anspruch 1, **dadurch gekennzeichnet, dass** in dem Blendenkörper (6) mehrere, in Abstand zueinander liegende Blendenöffnungen (7a, 7b) vorgesehen sind, und dass in den einzelnen Einstellpositionen der Blendenkörper (6) in verschiedenen Lagen am Sensorgehäuse (2) fixiert ist, so dass jeweils unterschiedliche Blendenöffnungen (7a, 7b) vor einer Durchtrittsfläche positioniert sind.

3. Ultraschallsensor nach Anspruch 2, **dadurch gekennzeichnet, dass** der Blendenkörper (6) in unterschiedlichen Lagen auf dem Sensorgehäuse (2) aufrastbar ist.

4. Ultraschallsensor nach Anspruch 1, **dadurch gekennzeichnet, dass** der Blendenkörper (6) nur eine Blendenöffnung (7) aufweist, deren Form und/oder Größe mittels eines Verstellmechanismus einstellbar ist.

5. Ultraschallsensor nach Anspruch 4, **dadurch gekennzeichnet, dass** der Blendenkörper (6) bei unterschiedlichen Einstellungen der Blendenöffnung (7) in unveränderter Lage am Sensorgehäuse (2) fixiert ist.

6. Ultraschallsensor nach einem der Ansprüche 4 oder 5, **dadurch gekennzeichnet, dass** mittels des Verstellmechanismus die Form und/oder Größe der Blendenöffnung (7) stufenlos einstellbar ist.

7. Ultraschallsensor nach einem der Ansprüche 4 bis 6, **dadurch gekennzeichnet, dass** im Blendenkörper (6) ein Blendenloch (12) eingearbeitet ist, welches zur Ausbildung einer einstellbaren Blendenöffnung (7) mit Blendenteilen (13a, 13b), die mittels des Verstellmechanismus einstellbar sind, mit variablem Überdeckungsgrad abdeckbar ist.

8. Ultraschallsensor nach Anspruch 7, **dadurch gekennzeichnet, dass** die Blendenteile (13a, 13b) mittels des Verstellmechanismus in gegenläufigen Richtungen verschiebbar gelagert sind.

9. Ultraschallsensor nach Anspruch 8, **dadurch gekennzeichnet, dass** der Verstellmechanismus zwei auf einer Spindel (15) angeordnete gegenläufige Gewinde zur Verstellung der Blendenteile (13a, 13b) aufweist.

10. Ultraschallsensor nach Anspruch 9, **dadurch gekennzeichnet, dass** der Verstellmechanismus eine Stellschraube (16) als Betätigungselement aufweist.

11. Ultraschallsensor nach einem der Ansprüche 1 bis 10, **dadurch gekennzeichnet, dass** das Sensorgehäuse (2) gabelförmig ausgebildet ist und zwei parallel in Abstand zueinander verlaufende Gabelarme (2a, 2b) aufweist, wobei in einem Gabelarm (2a) der Sender (4) und im anderen Gabelarm (2b) der Empfänger (5) angeordnet ist.

12. Ultraschallsensor nach einem der Ansprüche 1 bis 11, **dadurch gekennzeichnet, dass** die Objekte von auf einem Trägermaterial angeordneten Etiketten gebildet sind.

## Claims

1. Ultrasonic sensor (1) for detecting objects in a monitoring region, with a sensor housing (2), a transmitter (4) which emits ultrasonic waves (3), which are guided into the monitoring region via a first passage area of the sensor housing (2), a receiver (5) which receives ultrasonic waves (3), which - arriving from the monitoring region - are guided via a second passage area of the sensor housing (2), and with an evaluating unit for generating an object detection signal in dependence on the received signals of the receiver (5), wherein the object detection takes place according to the transmission principle, **characterised in that** at least one aperture body (6) is mountable in different setting positions at the sensor housing (2), wherein in the individual setting positions different aperture openings (7, 7a, 7b) of the aperture body (6) are positioned in front of a passage area.

2. Ultrasonic sensor according to claim 1, **characterised in that** a plurality of mutually spaced-apart aperture openings (7a, 7b) is provided in the aperture body (6) and that in the individual setting positions the aperture body (6) is fixed in different positions at the sensor housing (2) so that respectively different aperture openings (7a, 7b) are positioned in front of a passage area.

3. Ultrasonic sensor according to claim 2, **characterised in that** the aperture body (6) is detentable in different positions on the sensor housing (2).

4. Ultrasonic sensor according to claim 1, **characterised in that** the aperture body (6) has only one aperture opening (7), the shape and/or size of which is or are settable by means of an adjusting mechanism.

5. Ultrasonic sensor according to claim 4, **characterised in that** the aperture body (6) in the case of different settings of the aperture opening (7) is fixed in unchanged position at the sensor housing (2).

6. Ultrasonic sensor according to one of claims 4 and 5, **characterised in that** the shape and/or size of the aperture opening (7) is or are steplessly settable by means of the adjusting mechanism.

7. Ultrasonic sensor according to any one of claims 4 to 6, **characterised in that** an aperture hole (12) is formed in the aperture body (6) and for formation of a settable aperture opening (7) is coverable by aperture parts (13a, 13b), which are settable by means of the adjusting mechanism, with a variable degree of coverage.

8. Ultrasonic sensor according to claim 7, **characterised in that** the aperture parts (13a, 13b) are mounted to be displaceable in opposite directions by means of the adjusting mechanism.

9. Ultrasonic sensor according to claim 8, **characterised in that** the adjusting mechanism comprises two threads of opposite sense, which are arranged on a spindle (15), for adjusting the aperture parts (13a, 13b).

10. Ultrasonic sensor according to claim 9, **characterised in that** the adjusting mechanism comprises a setting screw (16) as actuating element.

11. Ultrasonic sensor according to any one of claims 1 to 10, **characterised in that** the sensor (2) is of forked construction and has two fork arms (2a, 2b) extending parallelly at a spacing from one another, wherein the transmitter (4) is arranged in one fork arm (2a) and the receiver (5) is arranged in the other fork arm (2b).

12. Ultrasonic sensor according to any one of claims 1 to 11, **characterised in that** the objects are formed by labels arranged on a carrier material.

## Revendications

1. Capteur d'ultrasons (1) pour détecter des objets dans une zone de surveillance, comprenant un boîtier de capteur (2), un émetteur (4) qui émet des ondes ultrasonores (3) qui sont guidées dans la zone de surveillance via une première surface de passage du boîtier de capteur (2), un récepteur (5) qui reçoit des ondes ultrasonores (3) provenant de la zone de surveillance qui sont guidées via une deuxième surface de passage du boîtier de capteur (2), et une unité d'évaluation servant à générer un signal de détection d'objet en fonction des signaux de réception du récepteur (5), la détection d'objet ayant lieu selon le principe de la transmission, **caractérisé en ce qu'**au moins un corps de diaphragme (6) peut être placé dans différentes positions de réglage sur le boîtier de capteur (2), différentes ouvertures de diaphragme (7, 7a, 7b) du corps de diaphragme (6) étant positionnées devant une surface de passage dans les différentes positions de réglage.

2. Capteur d'ultrasons selon la revendication 1, **caractérisé en ce que** plusieurs ouvertures de diaphragme (7a, 7b) situées à distance les unes des autres sont prévues dans le corps de diaphragme (6), et que dans les différentes positions de réglage, le corps de diaphragme (6) est immobilisé dans différentes positions sur le boîtier de capteur (2), de sorte que des ouvertures de diaphragme (7a, 7b) différentes sont chaque fois positionnées devant une surface de passage.

3. Capteur d'ultrasons selon la revendication 2, **caractérisé en ce que** le corps de diaphragme (6) peut être enclenché dans différentes positions sur le boîtier de capteur (2).

4. Capteur d'ultrasons selon la revendication 1, **caractérisé en ce que** le corps de diaphragme (6) présente une seule ouverture de diaphragme (7) dont la forme et/ou la taille est réglable au moyen d'un mécanisme de réglage.

5. Capteur d'ultrasons selon la revendication 4, **caractérisé en ce que** le corps de diaphragme (6) est immobilisé dans une position inchangée sur le boîtier de capteur (2) dans des réglages différents de l'ouverture de diaphragme (7).

6. Capteur d'ultrasons selon une des revendications 4 ou 5, **caractérisé en ce que** la forme et/ou la taille de l'ouverture de diaphragme (7) est réglable de façon continue au moyen du mécanisme de réglage.

7. Capteur d'ultrasons selon une des revendications 4 à 6, **caractérisé en ce qu'**un trou de diaphragme (12) est pratiqué dans le corps de diaphragme (6), lequel peut être occulté avec un degré d'occultation variable par des éléments de diaphragme (13a, 13b) qui sont réglables au moyen du mécanisme de réglage pour former une ouverture de diaphragme (7) réglable.

8. Capteur d'ultrasons selon la revendication 7, **caractérisé en ce que** les éléments de diaphragme (13a, 13b) sont montés de manière à pouvoir coulisser dans des directions contraires au moyen du mécanisme de réglage.

9. Capteur d'ultrasons selon la revendication 8, **caractérisé en ce que** le mécanisme de réglage présente deux filetages en sens contraire disposés sur une broche (15) pour le réglage des éléments de diaphragme (13a, 13b).

10. Capteur d'ultrasons selon la revendication 9, **caractérisé en ce que** le mécanisme de réglage présente une vis de réglage (16) comme élément d'actionnement.

11. Capteur d'ultrasons selon une des revendications 1 à 10, **caractérisé en ce que** le boîtier de capteur (2) est réalisé en forme de fourche et présente deux bras de fourche (2a, 2b) qui s'étendent parallèlement à distance l'un de l'autre, l'émetteur (4) étant disposé dans un bras de fourche (2a) et le récepteur (5) dans l'autre bras de fourche (2b).

12. Capteur d'ultrasons selon une des revendications 1 à 11, **caractérisé en ce que** les objets sont formés par des étiquettes disposées sur un matériau de support.
